Europäisches Patentamt

European Patent Office

Office européen des brevets

(11) Veröffentlichungsnummer: **0 344 442 B1**

## EUROPÄISCHE PATENTSCHRIFT

(45) Veröffentlichungstag der Patentschrift: **01.09.93**

(51) Int. Cl.5: **B01F 17/00**, A61K 7/08, A61K 7/50, C11D 1/06

(21) Anmeldenummer: **89106988.2**

(22) Anmeldetag: **19.04.89**

(54) **Konzentrierte pumpbare Polyethercarboxylate.**

(30) Priorität: **01.06.88 DE 3818626**

(43) Veröffentlichungstag der Anmeldung:
**06.12.89 Patentblatt 89/49**

(45) Bekanntmachung des Hinweises auf die
Patenterteilung:
**01.09.93 Patentblatt 93/35**

(84) Benannte Vertragsstaaten:
**AT BE CH DE ES FR GB IT LI LU NL SE**

(56) Entgegenhaltungen:
EP-A- 0 072 600    EP-A- 0 106 018
EP-A- 0 177 071    EP-A- 0 236 677
FR-A- 1 548 244    FR-A- 2 233 391
FR-A- 2 338 326    GB-A- 2 169 613
GB-A- 2 178 753

SEIFEN-ÖLE-FETTE-WACHSE, Band 109, Nr.
13, August 1983, Seiten 353-355, Augsburg,
DE; N.A.I. VAN PAASSEN:
"Alkylethercarboxylate: Hautfreundliche
Rohstoffe für kosmetische Anwendungen"

(73) Patentinhaber: **HÜLS AKTIENGESELLSCHAFT**

**D-45764 Marl(DE)**

(72) Erfinder: **Ruback, Wulf, Dr.**
**Moltkestrasse 15 a**
**D-4350 Recklinghausen(DE)**

**Beschreibung**

Die Erfindung betrifft konzentrierte pumpbare Polyethercarboxylate der Struktur

$$R - O -(A - O)_n - CH_2 - COOM \qquad (I),$$

worin

R = Alkyl mit 8 bis 24 C-Atomen oder alkylsubstituiertes Aryl mit 9 bis 24 C-Atomen,

A = Alkylen mit 2 bis 5 C-Atomen,

n = 5 bis 20 und

M = Alkali, Erdalkali, Ammonium oder substituiertes Ammonium

ist, sowie ein Verfahren zur Herstellung dieser Produkte durch Neutralisation der entsprechenden Polyethercarbonsäuren.

Die Anforderungen an oberflächenaktive Substanzen, die in kosmetischen Formulierungen eingesetzt werden sollen, sind seit Jahren einem ständigen Wandel unterworfen. Mit zunehmender Anwendung von Shampoos, Dusch- und Schaumbädern wird die dermatologische Verträglichkeit von solchen oberflächenaktiven Verbindungen zum vordringlichen Kriterium für die Rohstoffauswahl.

Gegenüber Alkalisalzen von Fettsäuren, Alkylsulfaten und Alkylethersulfaten haben Salze von Polyethercarbonsäuren in den letzten Jahren an Bedeutung gewonnen. Sie haben sich nach van Paassen, Seifen-Öle-Fette-Wachse 109 (1983), 353, als besonders hautverträglich erwiesen. Sie sind deshalb insbesondere auch für kosmetische Anwendungen sehr gut geeignet.

Zur Herstellung von Polyethercarboxylaten setzt man im allgemeinen Polyether in Gegenwart von Natronlauge mit dem Natriumsalz der Chloressigsäure um. Dabei erhält man das Natriumsalz einer Polyethercarbonsäure, das als Rohprodukt vor allem mit den Ausgangsverbindungen verunreinigt ist. Es enthält außerdem Kochsalz und ist sehr hochviskos. Im Reaktionsprodukt kann man entsprechend EP-B-106 018 einen Carboxymethylierungsgrad definieren:

Mole erhaltenes Carboxylat x 100 / Mole eingesetzter Polyether

Für kosmetische Zwecke kommen in erster Linie Produkte mit einem Carboxymethylierungsgrad von über 90 Mol-% zum Einsatz.

Das störende Kochsalz kann man nach Kosswig, Ullmanns Enzyklopädie der technischen Chemie, 4. Auflage, Band 22 (1982), 469, durch Ansäuern abtrennen. Dabei scheiden sich die wasserunlöslichen Polyethercarbonsäuren als niedrigviskose organische Phase ab, während das Kochsalz in der wäßrigen Phase verbleibt. Nach Phasentrennung erhält man ein gereinigtes Produkt, das bei einem geringen Wassergehalt einen Gehalt an Aktivsubstanz (tensidaktiver Feststoffgehalt) von in der Regel über 90 Gew.-% aufweist.

Viele Verarbeiter sind jedoch nicht in der Lage, die aus transporttechnischer Sicht hervorragenden freien Säuren zu den Salzen zu neutralisieren, die allein in den Fertigprodukten zum Einsatz kommen. Diese Verarbeiter sind deshalb nur an den fertigen Polyethercarboxylaten interessiert.

Die üblicherweise verwendeten Natriumsalze der Polyethercarbonsäuren sind jedoch so hochviskos, daß sie bereits bei Gehalten an Aktivsubstanz von etwa 25 Gew.-% nicht mehr pumpbar sind und damit auch nicht mehr verarbeitet werden können. Übliche Shampoos weisen beispielsweise Gehalte an Aktivsubstanz von maximal 22 bis 23 Gew.-% auf. Niedrige Gehalte an Aktivsubstanz der verarbeitbaren Produkte verursachen aber hohe Distributionskosten und stehen einer breiteren Vermarktung entgegen.

In DE-A-16 17 072 werden Weichmacher und nichtionische Tenside Reinigungsmittelkonzentraten, konzentrierten anionischen Tensiden, zugesetzt, wodurch die Lösungsgeschwindigkeit in kaltem Nasser erhöht wird. Obwohl die Mischungen auch Alkohol enthalten können, wird die verbesserte Löslichkeit der anionischen Tenside jedoch dem Gehalt an Weichmachern und nichtionischen Tensiden zugeschrieben. Formulierungen mit Nonylphenolethoxylaten und Dibutylphthalat, wie sie hier überwiegend angegeben werden, haben aber im kosmetischen Bereich keine Verwendung gefunden.

Nach DE-C-30 32 061 kann man verpumpbare oberflächenaktive Mischungen auf der Basis von Polyethercarbonsäuren dadurch erhalten, daß man den rohen Polyethercarboxylaten bis zu 10 % Wasser zusetzt und mit vorzugsweise einer tensidischen Säure auf pH 4 bis 7 einstellt, wodurch die Polyethercarbonsäuren zumindest teilweise freigesetzt werden.

Für kosmetische Formulierungen stört hier der hohe Kochsalzgehalt, die Verwendung eines fremden Tensids und daß die ungereinigte rohe Reaktionsmischung eingesetzt wird. Außerdem betrifft das Patent vorzugsweise Produkte, deren Carboxymethylierungsgrad von etwa 65 Mol-% für den Einsatz in Kosmetika

EP 0 344 442 B1

zu niedrig ist.

Aufgabe der vorliegenden Erfindung ist es, für die Verwendung in Kosmetika gut geeignete konzentrierte pumpbare Polyethercarboxylate, die kein Kochsalz und keine Fremdsäuren enthalten, bereitzustellen und ein Verfahren zur Herstellung derartiger Produkte zu entwickeln.

Die Aufgabe wird erfindungsgemäß dadurch gelöst, daß man konzentrierte Polyethercarbonsäuren der Struktur

$$R - O - (A - O)_n - CH_2 - COOH \qquad (II),$$

worin

R = Alkyl mit 8 bis 24 C-Atomen oder alkylsubstituiertes Aryl mit 9 bis 24 C-Atomen,
A = Alkylen mit 2 bis 5 C-Atomen und
n = 5 bis 20 ist,

neutralisiert und vor, während oder nach der Neutralisation einen Alkohol zusetzt.

Vorzugsweise wird Alkohol in solchen Mengen zugesetzt, daß die Endmischung neben der Aktivsubstanz einen Alkoholgehalt von 0,5 bis 15 Gew.-% aufweist und der Rest Wasser ist. Dabei werden ein- bis dreiwertige Alkohole mit 1 bis 6 C-Atomen eingesetzt. Geeignete Alkohole sind beispielsweise Methanol, Ethanol, n-Propanol, i-Propanol, n-Butanol, i-Butanol, t-Butanol, Pentanole, Ethylenglykol, Propylenglykol, Neopentylglykol, 1,4-Butandiol, 1,6-Hexandiol oder Glycerin.

Besonders bevorzugt ist ein Alkoholgehalt von 0,5 bis 7 Gew.-%, wobei einwertige Alkohole Vorrang haben. Im besonderen werden Ethanol und i-Propanol wegen der geringen benötigten Menge und einer sehr guten Geruchskompatibilität bevorzugt.

Die Zugabetemperatur liegt unterhalb des Siedepunktes des Alkohols. Im allgemeinen wird Alkohol bei 20 bis 40 °C zugesetzt.

Um Pumpbarkeit zu erreichen, brauchen keine klaren Mischungen vorzuliegen. Die Produkte können ein trübes bis opaleszierendes Aussehen aufweisen. Als pumpbar werden im Sinne der vorliegenden Erfindung Produkte mit einer Viskosität bei 25 °C von unter 10 000 mPa s (D = 50 sec$^{-1}$) angesehen.

Wird neben der Pumpbarkeit zusätzlich eine gute Lagerstabilität gewünscht, sollte so viel Alkohol zugesetzt werden, daß eine zumindest fast klare Mischung erhalten wird.

Die Polyethercarboxylate haben einen Gehalt an Aktivsubstanz von über 70 Gew.-%. Der Gesamtfeststoffgehalt kann noch etwas höher liegen, wenn die Produkte noch Polyether enthalten. Die Produkte haben vorzugsweise einen Carboxymethylierungsgrad von über 90 Mol-%.

In den Polyethercarboxylaten der Struktur I ist R beispielsweise Decyl, Lauryl, Isotridecyl, Myristyl, Palmityl, Stearyl oder Nonylphenyl. Vorzugsweise steht R für Alkyl mit 10 bis 16 C-Atomen.

A ist vorzugsweise Ethylen oder Propylen, wobei der Etherteil der Struktur I auch Ethylen- und Propyleneinheiten aufweisen kann. In einer besonders bevorzugten Ausführungsform steht A für Ethylen.

Beispiele für M sind Kalium, Natrium, Ammonium, Magnesium oder substituiertes Ammonium, wie Trimethylammonium und Triethanolammonium. Vorzugsweise steht M für Alkali oder Ammonium. Dabei werden Natrium und Kalium ganz besonders bevorzugt.

Die erfindungsgemäßen Polyethercarboxylate haben einen hohen Gehalt an Aktivsubstanz und einen hohen Carboxymethylierungsgrad. Sie sind pumpbar und frei von Kochsalz. Sie enthalten keine störenden Fremdtenside und sind deshalb sehr gut für Kosmetika, wie Haut- und Haarpflegemittel, einsetzbar.

Die folgenden Beispiele sollen die Erfindung verdeutlichen.

Beispiele 1 bis 6, Vergleichsbeispiel A

In einem 500-ml-Kolben mit Rührer werden 400 g Lauryl-/Myristyldecaglykoletheressigsäure

$$C_{12}H_{25}/C_{14}H_{29} - O - (C_2H_4O)_{10} - CH_2 - COOH$$

(Carboxymethylierungsgrad: 92 Mol-%) mit einem $C_{12}/C_{14}$-Molverhältnis von 70 : 30 und einem Wassergehalt von 7 % mit 40 g 50%iger Natronlauge verrührt. Nach 30 min ist die exotherme Reaktion abgeklungen. Man setzt dann 13 g Alkohol zu. Die Polyethercarboxylate haben nun folgende Zusammensetzung:

3

| Trockengehalt | 85 % |
|---|---|
| Alkoholgehalt | 3 % |
| Wassergehalt | 12 % |
| pH-Wert | 6,2 - 6,3 |

Der Gehalt an Aktivsubstanz ist nur geringfügig niedriger als der Trockengehalt. Es werden die in Tabelle 1 angegebenen Alkohole eingesetzt. Die Viskosität der Polyethercarboxylate wird mit einem rechnergesteuerten Rotationsviskosimeter (Haake RV 12) bei 25 °C bestimmt.

Tabelle 1

| Beispiel | 3 % Alkohol | Viskosität mPa s (D = 50 sec$^{-1}$) |
|---|---|---|
| 1 | Methanol | 1 780 |
| 2 | Ethanol | 540 |
| 3 | n-Propanol | 405 |
| 4 | i-Propanol | 420 |
| 5 | n-Butanol | 2 230 |
| 6 | Ethylenglykol | 3 070 |
| A | Wasser | >100 000* |

* fest, gelig, nicht meßbar

Wird also Alkohol durch Wasser ersetzt (Vergleichsbeispiel A), wobei hier der Gesamtwassergehalt auf 15 % erhöht wird, steigt die Viskosität sehr stark an.

Beispiele 7 bis 13

Es werden 182 bis 191 g Polyetheressigsäure der Struktur

$$C_{12}H_{25}/C_{14}H_{29} - O - (C_2H_4O)_n - CH_2 - COOH$$

mit einem $C_{12}/C_{14}$-Molverhältnis von 70 : 30 mit 50%iger Natronlauge bis zu pH 6,5 neutralisiert. Durch Wasserzugabe wird dann ein Wassergehalt von 12 bis 15 % eingestellt. Anschließend wird so lange Ethanol zugesetzt, bis die Polyethercarboxylate eine Viskosität von 2 400 ± 200 mPa s (D = 50 sec$^{-1}$) aufweisen.

Tabelle 2

| Beispiel | n | g EtOH zugesetzt | Endprodukt | | |
|---|---|---|---|---|---|
| | | | % EtOH | % $H_2O$ | % Feststoff |
| 7 | 5 | 13,6 | 6 | 13,4 | 80,6 |
| 8 | 8 | 7,8 | 3,5 | 13,5 | 83,1 |
| 9 | 10 | 4,4 | 2 | 13,2 | 84,9 |
| 10 | 12 | 2,2 | 1 | 12,8 | 86,2 |
| 11 | 14 | 1,1 | 0,5 | 12,7 | 86,8 |
| 12 | 16 | 5,4 | 2,5 | 12,4 | 85,1 |
| 13 | 18 | 4,3 | 2 | 12,1 | 85,9 |
| EtOH = Ethanol | | | | | |

EP 0 344 442 B1

Die Produkte wurden 8 Monate bei im Mittel 18 °C gelagert, wobei Extremwerte von 8 und 35 °C erreicht wurden. Nach 8 Monaten hatten alle Produkte Aussehen und Viskosität unverändert beibehalten.

**Patentansprüche**

1. Verfahren zur Herstellung von konzentrierten pumpbaren Polyethercarboxylaten der Struktur

   $R - O - (A - O)_n - CH_2 - COOM$      (I)

   dadurch gekennzeichnet,
   daß man konzentrierte Polyethercarbonsäuren der Struktur

   $R - O - (A - O)_n - CH_2 - COOH$      (II),

   worin jeweils
   R =      Alkyl mit 8 bis 24 C-Atomen oder alkylsubstituiertes Aryl mit 9 bis 24 C-Atomen,
   A =      Alkylen mit 2 bis 5 C-Atomen,
   n =      5 bis 20 und
   M =      Alkali, Erdalkali, Ammonium oder substituiertes Ammonium ist,
   neutralisiert und vor, während oder nach der Neutralisation einen Alkohol zusetzt.

2. Verfahren nach Anspruch 1,
   dadurch gekennzeichnet,
   daß man, bezogen auf die Endmischung, 0,5 bis 15 Gew.-% ein- bis dreiwertigen Alkohol mit 1 bis 6 C-Atomen einsetzt.

3. Verfahren nach Anspruch 2,
   dadurch gekennzeichnet,
   daß man 0,5 bis 7 Gew.-% einwertigen Alkohol verwendet.

4. Konzentrierte pumpbare Polyethercarboxylate der Struktur I, worin R, A, n und M die in Anspruch 1 genannten Bedeutungen haben,
   dadurch gekennzeichnet,
   daß sie einen Gehalt an Aktivsubstanz von über 70 Gew.-% und 0,5 bis 15 Gew.-% ein- bis dreiwertigen Alkohol mit 1 bis 6 C-Atomen aufweisen und der Rest Wasser ist.

5. Produkte nach Anspruch 4,
   dadurch gekennzeichnet,
   daß R Alkyl mit 10 bis 16 C-Atomen ist.

6. Produkte nach Anspruch 4,
   dadurch gekennzeichnet,
   daß A Ethylen und/oder Propylen ist.

7. Produkte nach Anspruch 4,
   dadurch gekennzeichnet,
   daß M Alkali oder Ammonium ist.

8. Produkte nach Anspruch 4,
   dadurch gekennzeichnet,
   daß sie einen Carboxymethylierungsgrad von über 90 Mol-% aufweisen.

**Claims**

1. A process for the preparation of concentrated pumpable polyethercarboxylates of the structure

   $R - O - (A - O)_n - CH_2 - COOM$      (I)

5

characterized in that concentrated polyethercarboxylic acids of the structure

$$R - O - (A - O)_n - CH_2 - COOH \quad (II),$$

in which, in each case,

R is alkyl having 8 to 24 carbon atoms or alkyl-substituted aryl having 9 to 24 carbon atoms,
A is alkylene having 2 to 5 carbon atoms,
n is from 5 to 20 and
M is an alkali metal, alkaline earth metal, ammonium or substituted ammonium,

are neutralised, and an alcohol is added before, during or after the neutralisation.

**2.** A process as claimed in claim 1, characterised in that, based on the final mixture, from 0.5 to 15 % by weight of a monohydric to trihydric alcohol having 1 to 6 carbon atoms is employed.

**3.** A process according to claim 2, characterised in that from 0.5 to 7 % by weight of a monohydric alcohol is used.

**4.** A concentrated pumpable polyethercarboxylate of the structure I, in which R, A, n and M are as defined in claim 1, characterised in that it contains more than 70 % by weight of active substance and from 0.5 to 15 % by weight of a monohydric to trihydric alcohol having 1 to 6 carbon atoms, and the remainder is water.

**5.** A product according to claim 4, characterised in that R is alkyl having 10 to 16 carbon atoms.

**6.** A product according to claim 4, characterised in that A is ethylene and/or propylene.

**7.** A product according to claim 4, characterised in that M is an alkali metal or ammonium.

**8.** A product according to claim 4, characterised in that it has a degree of carboxymethylation of greater than 90 mol %.

**Revendications**

**1.** Procédé de préparation de poly-éther-carboxylates concentrés pompables, de structure :

$$R - O - (A - O)_n - CH_2 - COOM \quad (I),$$

caractérisé par le fait que l'on neutralise des acides poly-éther-carboxyliques concentrés de structure :

$$R - O - (A - O)_n - CH_2 - COOH \quad (II),$$

dans laquelle chaque fois

R est un alkyle comportant de 8 à 24 atomes de carbone ou un aryle substitué par un alkyle et comportant de 9 à 24 atomes de carbone,
A est un alkylène comportant de 2 à 5 atomes de carbone,
$\underline{n}$ a une valeur de 5 à 20, et
$\overline{M}$ est un métal alcalin, alcalino-terreux, de l'ammonium ou un ammonium substitué,

et que l'on ajoute un alcool avant, pendant ou après la neutralisation.

**2.** Procédé selon la revendication 1, caractérisé par le fait que l'on utilise, relativement au mélange final, de 0,5 à 15 % en poids d'un mono- à tri-alcool comportant de 1 à 6 atomes de carbone.

**3.** Procédé selon la revendication 2, caractérisé par le fait que l'on utilise de 0,5 à 7 % en poids d'un mono-alcool.

**4.** Poly-éther-carboxylates concentrés pompables de la structure I, dans laquelle R, A, $\underline{n}$ et M ont les significations indiquées dans la revendication 1,

6

caractérisés par le fait qu'ils présentent une teneur en substance active supérieure à 70 % en poids et de 0,5 à 15 % en poids d'un mono-alcool à tri-alcool comportant de 1 à 6 atomes de carbone, le reste étant de l'eau.

5. Produits selon la revendication 4,
caractérisés par le fait que R est un alkyle comportant de 10 à 16 atomes de carbone.

6. Produits selon la revendication 4,
caractérisés par le fait que A est l'éthylène et/ou le propylène.

7. Produits selon la revendication 4,
caractérisés par le fait que M est un métal alcalin ou de l'ammonium.

8. Produits selon la revendication 4,
caractérisés par le fait qu'ils présentent un degré de carboxy-méthylation supérieur à 90 mole-%.